## Europäisches Patentamt
## European Patent Office
## Office européen des brevets

(19)

(11) Veröffentlichungsnummer : **0 173 924**
**B1**

(12) **EUROPÄISCHE PATENTSCHRIFT**

(45) Veröffentlichungstag der Patentschrift :
03.01.90

(51) Int. Cl.⁵ : **C 12 N 1/06**, C 12 P 21/02

(21) Anmeldenummer : 85110599.9

(22) Anmeldetag : 23.08.85

(54) Neues mechanisches Aufschlussverfahren von Bakterienzellen zur Isolierung von rekombinant hergestellten Peptiden.

(30) Priorität : 01.09.84 DE 3432196

(43) Veröffentlichungstag der Anmeldung :
12.03.86 Patentblatt 86/11

(45) Bekanntmachung des Hinweises auf die Patenterteilung : 03.01.90 Patentblatt 90/01

(84) Benannte Vertragsstaaten :
AT BE CH DE FR GB IT LI LU NL SE

(56) Entgegenhaltungen :
EP—A— 0 052 861
WO—A—83 /024 61
GB—A— 1 432 039
CHEMICAL ABSTRACTS, Band 79, Nr. 21, 26. November 1973, Seite 272, Zusammenfassung Nr. 124689g, Columbus, Ohio, US; & JP-A-72 43 834 (KANEGAFU-CHI CHEMICAL INDUSTRY CO., LTD) 06-11-1972
CHEMICAL ABSTRACTS, Band 91, Nr. 23, 3. Dezember 1979, Seite 461, Zusammenfassung Nr. 191106a, Columbus, Ohio, US; J.S. ERICKSON et al.: "Purification of acid ethanol-extracted human lymphoid interferons by Blue Sepharose chromatography", & ANAL. BIOCHEM. 1979, 98(1), 214-18

(73) Patentinhaber : BOEHRINGER INGELHEIM INTERNATIONAL GmbH
Postfach 20
D-6507 Ingelheim am Rhein (DE)

(72) Erfinder : Adolf, Günter, Dr.
Johannagasse 20/7
A-1050 Wien (AT)
Erfinder : Bodo, Gerhard, Prof. Dr.
Belghofergasse 27
A-1120 Wien (AT)
Erfinder : Gibley, Wolfgang
Dammstrasse 20
A-2485 Wampersdorf (AT)
Erfinder : Konopitzky, Kurt, Dipl.-Ing.
Hütteldorferstrasse 141
A-1140 Wien (AT)

EP 0 173 924 B1

## Beschreibung

In der Literatur werden bereits mehrere Aufschlußverfahren für mikrobiologische Zellen beschrieben.

So wird in der japanischen Offenlegungsschrift Nr. 72/43.834 (siehe auch C. A. 79, 124689g (1973)) ein mikrobielles Aufschlußverfahren, insbesondere für Hefe, unter Verwendung einer Entspannungsdüse und in der britischen Patentschrift 1 432 039 ein 2-stufiges Aufschlußverfahren beschrieben, bei welchem die Zellwände der eingesetzten Zellen zuerst chemisch geschwächt werden, z. B. durch Behandeln mit einer Säure oder Base, und anschließend durch Mahlen mechanisch zerstört werden.

Ferner wird in der EP-A-0 052 861 ein chemisches Aufschlußverfahren von Bakterienzellen zur Isolierung von recombinant hergestelltem Interferon, insbesondere von IFN-α, beschrieben, welches durch folgende Verfahrensschritte gekennzeichnet ist :

1. Eine Bakteriensuspension, insbesondere eine E. coli-Suspension, deren Bakterien das gentechnologisch hergestellte IFN enthalten, wird zur Abtötung der Bakterien mittels einer Mineralsäure auf pH 2,0 bis 2,5 eingestellt.

2. Die abgetöteten Bakterienzellen werden abgetrennt.

3. Die erhaltene saure Biomasse wird in einem Puffer, z. B. in Tris(2-amino-2-hydroxymethyl-1,3-propandiol) + Natriumchlorid, resuspendiert und mittels Natronlauge auf pH 7.3 eingestellt, wobei die Bakterienzellwände zerstört werden.

4. Nach Abtrennen der Bakterienzellen wird das nunmehr in der Lösung enthaltene Interferon nach bekannten Methoden abgetrennt und weiter gereinigt.

Desweiteren ist bekannt, daß Polypeptide wie Interferone gegenüber mechanischen Einflüssen (siehe Proceedings of the Society for experimentel Biology and Medicine 146, 249-253 (1974)) wenig stabil sind. Derartige mechanische Einflüsse treten jedoch bei der mechanischen Zerstörung der Bakterienzellen durch die hierbei auftretenden Scherkräfte auf. Aus diesem Grunde ist auch wohl bis heute kein mechanisches Aufschlußverfahren von Bakterienzellen zur Isolierung von einem gentechnologisch gebildetem Polypeptid, z. B. von Interferon, bekannt geworden, bei welchem dieses in hohen Ausbeuten isoliert werden kann.

Der vorliegenden Erfindung liegt somit die Aufgabe zugrunde, ein verbessertes Extraktionsverfahren aus Bakterienzellen für recombinant hergestellte Polypeptide aufzufinden, bei welchem die Bakterienzellen mechanisch aufgeschlossen werden.

Erfindungsgemäß wird diese Aufgabe dadurch gelöst, daß nach Abtrennen der mittels einer Mineralsäure nach bekannten Methoden abgetöten Bakterien (siehe beispielsweise Sterilisation, Desinfektion, Konservierung, Chemotherapie — Verfahren, Wirkstoffe, Prüfungsverfahren von Dr.

K. H. Wallläußer und Prof. Dr. H. Schmidt, Georg Thieme Verlag, Stuttgart, 1967, Seiten 145-161), welche das recombinant hergestellte Polypeptid, z. B. ein säurestabiles Interferon, enthalten, die erhaltene Biomasse in einer wässrigen Säure bei einem pH von 2,0 bis 4,0, z. B. in einer Mineralsäure wie verdünnte Salzsäure oder in einer wässrigen Alkansäure wie Essigsäure oder Propionsäure resuspendiert wird, die Bakterienzellwände durch die auftretenden Scherkräfte mechanisch zerstört werden, z. B. mit Hilfe eines Homogenisators, vorzugsweise mit einem Homogenisator vom Typ des Ultra-Turrax, und anschließend nach Neutralisation der erhaltenen Suspension und Abtrennen der Bakterienbruchstücke das nunmehr sich in der Lösung befindliche Polypeptid nach bekannten Methoden abgetrennt und weiter gereinigt wird.

Zur Isolierung von einem rekombinant hergestellten säurestabilen Interferon, z. B. einem α-Interferon wie IFN-α2(arg), wird das erfindungsgemäße Verfahren besonders vorteilhaft bei einem pH von 2,5 bis 3,5, vorzugsweise in verdünnter Salzsäure von pH 2,5 oder in 1 %iger Essigsäure, durchgeführt.

Die Drehzahl des Homogenisators und die erforderliche Zeit, um die Bakterienzellen aufzuschließen, hängt hierbei von der Dichte der eingesetzten Biomasse ab. Weist beispielsweise die Biomasse eine Dichte von 1,03 bis 1,05 auf, so liegt die Drehzahl des Homogenisators zweckmäßigerweise zwischen 5 000 und 10 000 U/Min. Bei einer Dichte der Biomasse von beispielsweise 1,04 liegt die Drehzahl des Homogenisators vorzugsweise zwischen 7 000 und 8 000 U/Min. und die Aufschlußzeit bei 2 Minuten.

Desweiteren kann es in manchen Fällen von Vorteil sein, wenn nach der Homogenisation vor der Neutralisation ein Fällungsmittel, z. B. Polyäthylenimin in einer Konzentration von 0,1-0,5 %, zugesetzt wird.

Am besten läßt sich das erfindungsgemäße Verfahren im Labor-Maßstab mit einem IKA-Ultra-Turrax T45 und im größeren Maßstab mit dem Dispax-Reactor 3-6/6 jeweils der Firma Janke & Kunkel KG (D-7813 Staufen) durchführen. Hierbei liegt die Drehzahl ungefähr zwischen 5 000 und 10 000 U/Min. und die Aufschlußzeit zwischen 1 und 10 Minuten.

Der Ultra-Turrax T45 ist ein Dispergiergerät, bei dem ein Messer mit hoher Umdrehungszahl gegen einen Spaltenkranz rotiert und somit die Bakterienzellen in einem hochturbulenten Feld zerschlägt. Zur Zerstörung der Bakterienzellen wird das Gerät in ein Becherglas mit dem zu homogenisierenden Medium gehalten, wobei dieses durch einen Pumpeneffekt umgewälzt wird.

Demgegenüber ist der Homogenisator Dispax-Reactor 3-6/6 ein dreistufiger Ultra-Turrax mit Zwangsführung, das heißt, es müssen alle Bakterienzellen die Homogenisierkammern mit der gleichen Durchflußgeschwindigkeit durchwandern.

Hierdurch wird ein gleichmäßig aufgeschlossenes Bakterienhomogenisat erhalten. In diesem Dispax-Reactor beträgt die Drehzahl 3.000 bis 8.000 U/Min. und der Förderdruck maximal ca. 2 bar am Ablauf. Mit diesem Homogenisator können 50 l Biomasse innerhalb von 1 bis 10 Minuten homogenisiert werden.

Figur I stellt einen Querschnitt durch einen Homogenisator nach Art des Dispax-Reactors 3-6/6 dar. In dieser Figur ist F die Zulaufkammer für das zu homogenisierende Füllgut, E eine Gleitringdichtung an der Antriebsstelle H ; G stellt das Hauptlager dieser Antriebswelle dar. Auf der Antriebswelle H sitzen in Reihe zueinander drei zylinderförmige Rotoren C, die im Eingriff zu entsprechend ausgestellten Statoren D stehen, welche an den Innenwänden des Homogenisator-Gehäuses befestigt sind. Am Ende der Antriebswelle H ist eine Förderschnecke B befestigt, die in den Ablauf A hineinragt. Zur Kühlung der Statoren dient eine sich um die Statoren erstreckende Kühlkammer mit Einlaß L und Auslaß K für die Kühlwasserführung. Die Gleitringdichtung am Hauptlager wird über den Wassereinlauf J mit Kühlwasser, vorzugsweise unter erhöhtem Druck, beispielsweise 1,5 bar, versorgt. Die zylinderförmigen Rotoren können als Doppel-Trommeln oder Dreifach- bzw. Mehrfach-Trommeln ausgebildet sein, ihre Trommelwände ragen in die Zwischenräume entsprechender Gegentrommelwände der Statoren hinein. Die zu homogenisierende Flüssigkeit wird, angetrieben durch die Schnecke B, durch die Zwischenräume zwischen den Rotoren und Statoren gezogen, durch die rasche Rotation der Rotoren werden die Bakterienzellwände durch die auftretenden Scherkräfte zerstört, das so homogenisierte Gut verläßt den Homogenisator durch den Ablauf A.

Prinzipiell ist das Verfahren auch mit anderen bekannten mechanischen Zellaufschlußverfahren durchführbar wie sie beispielsweise in Biochemical Engineering, Seiter 358ff (Second Edition, Academic Press 1973) beschrieben sind. Die Ausbeuten sind jedoch dann niedriger, während man mit dem erfindungsgemäßen Verfahren unter Verwendung von Ultra Turrax T45 oder vom Dispax-Reactor 3-6/6 eine höhere Ausbeute, beispielsweise an Interferon, bezogen auf die eingesetzte Biomasse erhält als mit den bisher bekannten Verfahren, was auf Grund des oben angeführten Standes der Technik nicht vorhersehbar war.

Die nachfolgenden Beispiele sollen die Erfindung näher erläutern :

Beispiel 1

Eine Bakteriensuspension des E. Coli-Klons HB 101/pER 33 (siehe EP-A-0 115 613), deren Bakterien das Human-Interferon Alpha 2 enthalten, wird zur Abtötung der Bakterien mittels Mineralsäuren auf pH 2,0 bis 2,5 eingestellt. Die abgetöteten Bakterienzellen werden aus der Suspension abgetrennt und bei — 20 °C tiefgefroren.

20 kg einer tiefgefrorenen Biomasse der Charge A wurden in 250 l einer 1 %igen Essigsäure bei 8 °C aufgeschlämmt. Nach dem vollständigen Auftauen der Biomasse wurde mit dem dreistufigen Homogenisator (Dispax-Reactor 3-6/6) dispergiert. Zum feindispergierten Lysat wurde als Fällungshilfsmittel 0,1 %iges Polyäthylenimin zugesetzt. Dann erfolgte die pH-Wert-Änderung mit 5n Natronlauge auf pH 7,5. Nach zweistündiger Extraktionszeit wurde die ausgelaugte Biomasse mittels Tellerseperator (Westfalia) bei der Durchflußrate von 1,5 l/min abgetrennt. Von der Kläre wurden Proben zur Bestimmung des Interferon- und Proteingehaltes gezogen und ausgetestet.

Ausbeute : $46,9 \times 10^6$ I.E./g Biomasse.

Um den Einfluß des Homogenisators auf die Interferon-Ausbeute beurteilen zu können, wurden 500 ml der aufgeschlämmten Biomasse vor dem Dispergieren entnommen und ohne Homogenisationsschritt nach EP-A-0 052 861 extrahiert. Die pH-Wert-Änderung auf 7,3 erfolgte mit 3n Natronlauge. Nach einstündiger Extraktionszeit wurde das Lysat von der ausgelaugten Biomasse mit einer Laborzentrifuge geklärt. Vom Überstand wurden wie oben. Proben für Interferon- und Proteingehalt gezogen und getestet.

Ausbeute nach EP-A-0 052 861 : $25,3 \times 10^6$ I.E./g Biomasse.

Beispiel 2

Hergestellt analog Beispiel 1 unter Verwendung der Biomasse der Charge B.

Ausbeute : $21,3 \times 10^6$ I.E./g Biomasse.

Ausbeute nach EP-A-0 052 861 : $9,7 \times 10^6$ I.G./g Biomasse.

Beispiel 3

Hergestellt analog Beispiel 1 unter Verwendung der Biomasse der Charge C.

Ausbeute : $13,8 \times 10^6$ I.E./g Biomasse.

Ausbeute nach EP-A-0 052 861 : $5,1 \times 10^6$ I.E./g Biomasse.

Beispiel 4

Hergestellt analog Beispiel 1 unter Verwendung von 1 000 g Biomasse der Charge BC24, welche in 10 000 ml verdünnter Salzsäure (pH 2,5 ; 4 ml 37 %ige Salzsäure mit Wasser auf 10.000 ml aufgefüllt) aufgenommen und mit dem IKA-Ultra-Turrax T45 dispergiert wurde.

Ausbeute : $50 \times 10^6$ I.E./g Biomasse.

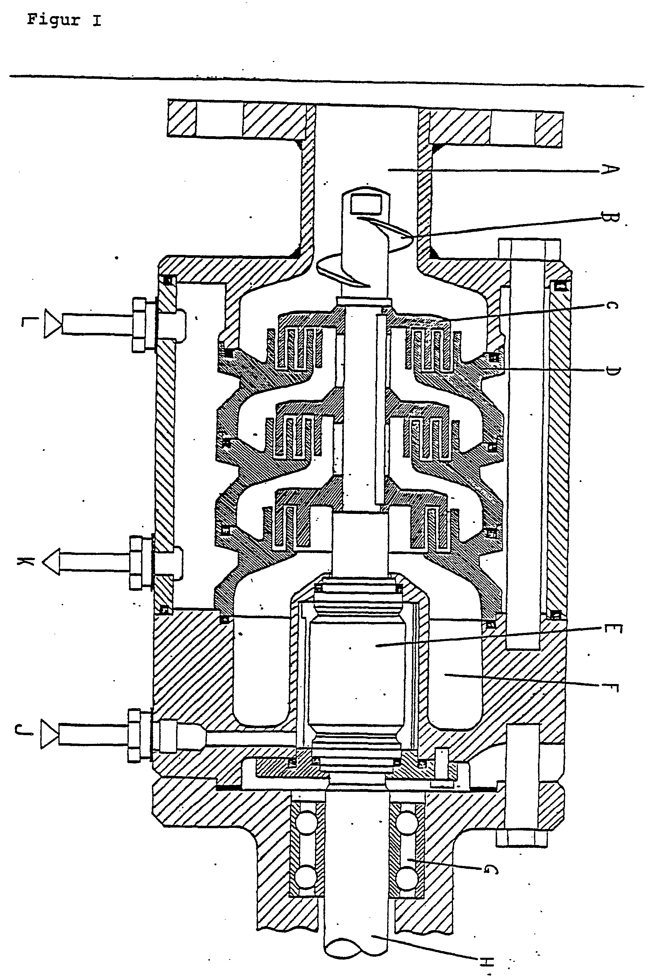

**Patentansprüche**

1. Verfahren zur Isolierung von einem recombinant hergestelltem Polypeptid aus Bakterienzellen, dadurch gekennzeichnet, daß Bakterienzellen, welche ein recombinant hergestelltes Polypeptid enthalten, suspendiert in einer wässrigen Säure bei einem pH von 2,0 bis 4,0 mittels Scherkräften mechanisch zerstört werden und anschließend, das nunmehr in der erhaltenen Suspension befindliche Polypeptid nach bekannten Methoden abgetrennt und weiter gereinigt wird.

2. Verfahren gemäß Anspruch 1, dadurch gekennzeichnet, daß Bakterienzellen, welche ein rekombinant hergestelltes säurestabiles Interferon enthalten, eingesetzt werden.

3. Verfahren gemäß Anspruch 1, dadurch gekennzeichnet, daß Bakterienzellen, welche ein rekombinant hergestelltes α-Interferon enthalten, eingesetzt werden.

4. Verfahren gemäß Anspruch 1, dadurch gekennzeichnet, daß Bakterienzellen, die recombinant hergestelltes IFN-α2(arg) enthalten, eingesetzt werden.

5. Verfahren gemäß den Ansprüchen 2 bis 4, dadurch gekennzeichnet, daß dieses bei einem pH von 2,5 bis 3,5 durchgeführt wird.

6. Verfahren gemäß den Ansprüchen 1 bis 5, dadurch gekennzeichnet, daß die Bakterienzellen mit einem Homogenisator zerstört werden.

7. Verfahren gemäß den Ansprüchen 1 bis 6, dadurch gekennzeichnet, daß als Säure eine Mineralsäure oder eine Alkansäure wie Essigsäure oder Propionsäure verwendet wird.

8. Verfahren gemäß den Ansprüchen 1 bis 7, dadurch gekennzeichnet, daß als wässrige Säure verdünnte Salzsäure (pH 2,5) oder 1 %ige Essigsäure verwendet wird.

## Claims

1. Process for isolating a polypeptide prepared by recombinant methods from bacterial cells, characterised in that bacterial cells containing a polypeptide prepared by genetic engineering, these cells being suspended in an aqueous acid at a pH of from 2.0 to 4.0, are mechanically destroyed by shear forces and subsequently the polypeptide now present in the resulting suspension is separated and further purified by known methods.

2. Process as claimed in claim 1, characterised in that bacterial cells are used which contain an acid-stable interferon prepared by recombinant methods.

3. Process as claimed in claim 1, characterised in that bacterial cells containing an α-interferon prepared by recombinant methods are used.

4. Process as claimed in claim 1, characterised in that bacterial cells containing IFN-α2(arg) prepared by recombinant methods are used.

5. Process as claimed in claims 2 to 4, characterised in that it is carried out at a pH of from 2.5 to 3.5.

6. Process as claimed in claims 1 to 5, characterised in that the bacterial cells are destroyed using a homogeniser.

7. Process as claimed in claims 1 to 6, characterised in that the acid used is an inorganic acid or an alkanoic acid such as acetic or propionic acid.

8. Process as claimed in claims 1 to 7, characterised in that the aqueous acid used is dilute hydrochloric acid (pH 2.5) or 1 % acetic acid.

## Revendications

1. Procédé pour l'isolement d'un polypeptide préparé par des techniques recombinantes à partir de cellules bactériennes, caractérisé en ce que des cellules bactériennes qui contiennent un polypeptide préparé par des techniques recombinantes, mises en suspension dans un acide aqueux à un pH de 2,0 jusqu'à 4,0 sont détruites mécaniquement au moyen de forces de cisaillement et subséquemment le polypeptide se trouvant à présent dans la suspension obtenue est séparé selon des méthodes connues et soumis à une purification ultérieure.

2. Procédé selon la revendication 1, caractérisé en ce que l'on met en œuvre des cellules bactériennes qui contiennent un interféron stable en milieu acide, préparé par des techniques recombinantes.

3. Procédé selon la revendication 1, caractérisé en ce que l'on met en œuvre des cellules bactériennes qui contiennent un interféron α préparé par des techniques recombinantes.

4. Procédé selon la revendication 1, caractérisé en ce que l'on met en œuvre des cellules bactériennes qui contiennent de l'IFN-α2(arg) préparé par des techniques recombinantes.

5. Procédé selon les revendications 2 à 4, caractérisé en ce que celui-ci est réalisé à un pH de 2,5 jusqu'à 3,5.

6. Procédé selon les revendications 1 à 5, caractérisé en ce que les cellules bactériennes sont détruites à l'aide d'un homogénéisateur.

7. Procédé selon les revendications 1 à 6, caractérisé en ce qu'en tant qu'acide on utilise un acide minéral ou un acide alcanoïque tel que l'acide acétique ou l'acide propionique.

8. Procédé selon les revendications 1 à 7, caractérisé en ce qu'en tant qu'acide aqueux on utilise de l'acide chlorhydrihque dilué (pH 2,5) ou de l'acide acétique à 1 %.

Figur I